(19) **Europäisches Patentamt / European Patent Office / Office européen des brevets**

(11) **EP 4 272 755 A1**

(12) **EUROPEAN PATENT APPLICATION**
published in accordance with Art. 153(4) EPC

(43) Date of publication:
**08.11.2023 Bulletin 2023/45**

(21) Application number: **20967606.3**

(22) Date of filing: **30.12.2020**

(51) International Patent Classification (IPC):
**A61K 39/125** (2006.01)      **A61K 39/39** (2006.01)
**A61P 31/14** (2006.01)

(52) Cooperative Patent Classification (CPC):
**A61K 39/125; A61K 39/39; A61P 31/14**

(86) International application number:
**PCT/CN2020/141693**

(87) International publication number:
**WO 2022/141266 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Sinovac Biotech Co., Ltd.**
**Beijing 100085 (CN)**

(72) Inventors:
• **LI, Yajing**
  **Beijing 100085 (CN)**
• **SHEN, Huan**
  **Beijing 100085 (CN)**

• **YIN, Yanhui**
  **Beijing 100085 (CN)**
• **JI, Wei**
  **Beijing 100085 (CN)**
• **SONG, Meng**
  **Beijing 100085 (CN)**
• **GAO, Qiang**
  **Beijing 100085 (CN)**
• **YIN, Weidong**
  **Beijing 100085 (CN)**

(74) Representative: **Kilger, Ute**
**Boehmert & Boehmert**
**Anwaltspartnerschaft mbB**
**Pettenkoferstrasse 22**
**80336 München (DE)**

(54) **COMBINED VACCINE FOR PREVENTING HAND, FOOT AND MOUTH DISEASE, PREPARATION METHOD THEREFOR AND USE THEREOF**

(57)   Disclosed is a combination vaccine for preventing HFMD, comprising inactivated Enterovirus type 71, and inactivated Coxsackievirus A group type 16, type 10 and type 6. Also disclosed is the method for the preparation of the combination vaccine. The adsorption effect and stability of the prepared vaccine are good. The above antigens do not interfere with each other's antigenicity and immune effect after immunizing the subject, and have good immunogenicity and safety. The application of the combination vaccine can significantly simplify the vaccination process, improve vaccination efficiency and reduce costs.

## Description

### Technical Field

The present invention relates to the biotechnical field, in particularly to a combination vaccine for preventing HFMD, preparation method and use thereof.

### Background

HFMD (hand-foot-mouth disease) is listed as a Class C infectious disease in "Law on the Prevention and Control of Infectious Diseases" in China. It is an acute infectious disease caused by a variety of enteroviruses, and is more common in infants and young children. HFMD is prevalent in summer, with a high incidence among preschool children, and adults may be the indirect source of infection. It is mainly manifested as rash on the mouth, hands and feet, and can be complicated by meningitis, encephalitis, pulmonary edema, circulatory failure and other severe diseases that may lead to death. In addition to the common Enterovirus type 71 (EV71) and Coxsackievirus A16 (CA16), the viruses that cause HFMD further include Coxsackievirus A group (CA) types 2, 4, 5, 6, 10, etc., Coxsackievirus B group (CB) types 1, 2, 3, 4, 5, etc., Enterovirus type 68 (EV68) and Echovirus (Echo), etc. While EV71 and CA16 are the main pathogens causing HFMD outbreaks, reports of HFMD outbreaks caused by CA10 and CA6 have gradually increased in recent years, and they have even become the main epidemic serotypes in some regions.

HFMD has become one of the public health problems that seriously threatens children's health and social stability. At present, there is no effective antiviral drug for the treatment of HFMD clinically. The approved EV71 inactivated vaccine, though is on the market, cannot provide protection against other types of HFMD. And due to the change of pathogenic spectrum of enterovirus of HFMD, the wide variety of main strains leading to the high incidence of HFMD, and lack of cross-immune protection between virus strains, it is challenging to prevent and control HFMD. It is difficult to control the outbreak and epidemic of HFMD caused by CA16, CA10 and CA6 by EV71 vaccination. Therefore, there is an urgent need to prepare corresponding multivalent vaccines and multi-vaccines to cope with HFMD outbreaks and epidemics of severe diseases. At present, there are no reports on the development of quadrivalent combination vaccines of EV71, CA16, CA10 and CA6. Firstly, HFMD caused by viruses of EV71, CA16, CA10 and CA6 has recently occurred in a large outbreak in a relatively short period of time; secondly, HFMD caused by viruses has a certain geographical distribution, and for developed countries with relatively low incidence, there are fewer institutions investing in relevant research and development; and thirdly, the immune interference among different virus antigens is unknown, and this interference may directly reduce the immunogenicity of a single component of the vaccine and affect the immune effect. Therefore, in order to protect the body against a series of viruses causing HFMD on a larger scale, it is imperative to establish a comprehensive HFMD prevention and control system in China and expand protection through the preparation of combination vaccines.

At present, EV71 vaccine has been available. According to clinical research data, the use of the vaccine has a significant effect on the prevention of HFMD caused by EV71, especially for severe diseases, but EV71 vaccine has weak cross-protection against coxsackievirus. In view of the trend of enterovirus epidemic serotypes, there is an urgent need for a multi-serotype combination vaccine that can prevent HFMD in a wider range.

### Summary of the Invention

The object of the present invention is to provide a combination vaccine for preventing HFMD.

In particularly, the present invention provides a combination vaccine for preventing HFMD, wherein the combination vaccine comprises inactivated Enterovirus type 71, and inactivated Coxsackievirus A group type 16, type 10 and type 6.

Wherein, a strain of the Enterovirus type 71 is preferable a strain with the deposit number of CGMCC No.3544.

The inactivated Coxsackievirus A group type 16, type 10 and type 6 according to the present invention are all newly found strains of the present invention, and have excellent immunogenicity. The new strains according to the present invention are deposited in China General Microbiological Culture Collection Center (CGMCC) on December 18, 2019. In this regard, the deposit number of Coxsackievirus A group type 16 strain is CGMCC No. 18886, the deposit number of Coxsackievirus A group type 10 strain is CGMCC No. 18887, and the deposit number of Coxsackievirus A group type 6 strain is CGMCC No. 18888.

A content of antigen of the inactivated Enterovirus type 71 in the combination vaccine according to the present invention is 100~1000U/mL.

A content of antigen of the inactivated Coxsackievirus A group type 16 in the combination vaccine according to the present invention is 200~3000U/mL, preferably is 200-1600U/mL.

A content of antigen of the inactivated Coxsackievirus A group type 10 in the combination vaccine according to the present invention is 200~3000U/mL, preferably is 200-1600U/mL.

A content of antigen of the inactivated Coxsackievirus A group type 6 in the combination vaccine according to the present invention is 1000~3000U/mL.

In an embodiment of the present invention, the combination vaccine further comprises an aluminum adjuvant.

In an embodiment of the present invention, the aluminum adjuvant is selected from the group consisting of aluminum hydroxide, aluminum phosphate and aluminum sulfate.

In an embodiment of the present invention, the final concentration of the aluminum adjuvant in aluminum content calculated by aluminum ion is 0.1-1.0 mg/mL, preferably is 0.2-0.8 mg/mL.

The combination vaccine according the present invention is preferably in liquid form.

The present invention further provides a method for the preparation of the combination vaccine, comprising the following steps: preparing a stock solution for each of the viruses respectively, using an aluminum adjuvant to adsorb each of the stock solutions respectively to provide a virus aluminum adsorption product, and then mixing the aluminum adsorption products to provide the combination vaccine.

The present invention further provides a use of the combination vaccine in the preparation of a medicament for the prevention or treatment of a disease caused by Enterovirus type 71, Coxsackievirus A group type 16, Coxsackievirus A group type 10, and/or Coxsackievirus A group type 6.

The combination vaccine according to the present invention relates to a combination of four common serotypes (EV71, CA16, CA10, CA6) causing HFMD, which can protect a person from HFMD caused by EV71, CA16, CA10 and CA6 in a wider range.

Among the four serotypes of viruses involved in the present invention, CA16, CA10 and CA6 were independently isolated by the present inventor in the laboratory, and EV71 was isolated by the China CDC (Chinese Center for Disease Control and Prevention) and identified by the Spot-picking & Bank-constructing Group of Sinovac Biotech Co.,Ltd. The isolation method is as follows: diluting and filtrating specimens collected from throat swabs or feces of clinical patients, inoculating cells, isolating pure single virus via spot picking, purifying, screening and identification; seeding and expanding cells to construct a virus strain library. The whole tests and stability of the virus strains were studied in accordance with the relevant requirements of the 2015 edition of the Pharmacopoeia of the People's Republic of China.

In an embodiment of the present invention, the combination vaccine according to the present invention is prepared by the following steps:

Preparation method of EV71 stock solution: Vero cells were inoculated with EV71 virus in a certain MOI (multiplicity of infection), and cultured in a microcarrier fermenter to scale up step by step or by cell factory plane culturing for 5-9 days; and the virus suspension was harvested. The virus suspension was inactivated by formaldehyde, and was ultrafiltered and concentrated through a 100-500KD ultrafiltration membrane package. The ultrafiltered and concentrated virus suspension was treated by sucrose density gradient centrifugation for purification and separating hollow and solid virus particles. The target tube virus was collected and desugared by ultrafiltration. The desugared virus suspension was treated with a non-restriction endonuclease, and was ultrafiltered to remove the host cell DNA, the impurity residues such as endonuclease were removed by molecular sieve chromatography, and EV71 virus stock solution was obtained after the above steps.

Preparation method of CA16 stock solution: Vero cells were inoculated with CA16 virus in a certain MOI (multiplicity of infection), and cultured in a microcarrier fermenter to scale up step by step or by cell factory plane culturing for 4-9 days; and the virus suspension was harvested. The virus suspension was cleared, ultrafiltered and concentrated through a 100-500KD ultrafiltration membrane package. The ultrafiltered and concentrated virus suspension was treated by sucrose density gradient centrifugation for purification to remove the impurity and separating hollow and solid virus particles. The ultrafiltered and centrifugated target tube virus was collected and desugared by ultrafiltration. The desugared virus suspension was treated with a non-restriction endonuclease, and was ultrafiltered to remove the host cell DNA, then inactivated by formaldehyde, and CA16 virus stock solution was obtained after the above steps.

Preparation method of CA10 stock solution: Vero cells were inoculated with CA10 virus in a certain MOI (multiplicity of infection), and cultured in a microcarrier fermenter to scale up step by step or by cell factory plane culturing for 4-9 days; and the virus suspension was harvested. The virus suspension was cleared, ultrafiltered and concentrated through a 100-500KD ultrafiltration membrane package. The ultrafiltered and concentrated virus suspension was treated by sucrose density gradient centrifugation for purification to remove the impurity and separating hollow and solid virus particles. The ultrafiltered and centrifugated target tube virus was collected and desugared by ultrafiltration. The desugared virus suspension was treated with a non-restriction endonuclease, and was ultrafiltered to remove the host cell DNA, then inactivated by formaldehyde, and CA10 virus stock solution was obtained after the above steps.

Preparation method of CA6 stock solution: Vero cells were inoculated with CA6 virus in a certain MOI (multiplicity of infection), and cultured in a microcarrier fermenter to scale up step by step or by cell factory plane culturing for 4-9 days; and the virus suspension was harvested. The virus suspension was cleared, ultrafiltered and concentrated through a 100-500KD ultrafiltration membrane package. The ultrafiltered and concentrated virus suspension was treated by sucrose density gradient centrifugation for purification to remove the impurity and separating hollow and solid virus particles. The ultrafiltered and centrifugated target tube virus was collected and desugared by ultrafiltration. The desugared virus suspension was treated with a non-restriction endonuclease, and was ultrafiltered to remove the host cell DNA, then inactivated by formaldehyde, and CA6 virus stock solution was obtained after the above steps.

The purification process of virus suspension was carried out by physical methods completely, which effectively removed impurity residues such as host protein, host DNA, and endonuclease, and thus provided a guarantee for the safety of the vaccine.

The stock solutions of the four serotypes were adsorbed with an aluminum adjuvant respectively and mixed in a certain proportion to prepare a semi-finished vaccine. The finished vaccine was prepared after packaging.

**Beneficial effects of the present invention**

The combination vaccine according to the present invention has good process consistency and stable process. The best inactivation process parameters are selected after conducting a large number of experiments, thus the finished product has a good stability. It has been proved by animal experiments that the vaccine has good safety and effectiveness.

The combination vaccine according to the present invention comprises the main pathogens of HFMD, and can prevent the occurrence of HFMD caused by viruses of EV71, CA16, CA10 and CA6. The present invention shows that the above antigens do not interfere with each other's antigenicity and immune effect after immunizing the recipients, and have good immunogenicity and safety.

The combination vaccine according to the present invention can simultaneously prevent the infection of multiple pathogens, and there is no mutual interference among these antigens, and the corresponding immunogenicity will not be reduced compared with the immunogenicity of individual antigens. The application of the combination vaccine can significantly simplify the vaccination process, improve the vaccination efficiency and reduce costs, which is the general trend of future technology development and also the future market demand.

**Detailed Description of the Present Invention**

The present invention is further described by the examples below. It should be understood that the examples of the present invention only intend to illustrate the present invention, but not to limit the present invention. Under the concept of the present invention, any simple improvements to the present invention fall within the protection scope of the present invention.

**1. Preparation of finished combination vaccine**

**(1) Separation of virus strains**

Throat swabs or feces of HFMD patients were obtained from local centers for disease control and prevention, diluted with normal saline, centrifuged, filtered and sterilized by filters of $0.45\mu m$ and $0.22\mu m$, respectively. Sensitive cells (Vero cells or human diploid cells) that had good cell growth status and converged to a monolayer were selected. The virus culture medium was Solution 199 containing 2% fetal bovine serum. The treated throat swabs/feces specimen was inoculated in a certain proportion, and then placed in an incubator under 5% $CO_2$ at 32.0°C~36.5°C for stationary culture. After inoculation, the cells were observed daily to see whether the characteristic enterovirus cytopathic effect (CPE) appeared. If CPE appeared and the CPE degree was more than +++, the cell culture was harvested and then passaged twice. If no CPE appeared, the cell culture was cultured until day 7, freeze-thaw once, and then harvested and continued to passage twice. One cell culture tube with cells in good growth status and no specimen inoculation was used as a cell control. The cell control should be free of CPE to establish the test.

After three blind passages, the specimens with characteristic enterovirus cytopathic effect (CPE) were judged to be positive for virus isolation, and PCR identification for the cell cultures with positive virus isolation was conducted.

**(2) Identification of the strains**

ELISA enzyme-linked immunoassay and PCR were used to identify the isolated strain at the immunological and molecular levels, respectively.

EV71 antigen detection system: the plate was coated with rabbit anti-EV71 polyclonal antibody, placed at 2-8°C overnight, blocked for 1-2 hours with 10% fetal bovine serum at 37°C, and added with virus culture that to be identified. A negative control well was set up. The plate was then incubated at 36~37°C for 1 hour, washed for 3~5 times, added with an EV71 specific monoclonal antibody, incubated at 36~37°C for 1 hour, washed for 3~5 times, and colored, and then the reaction was terminated.

CA16 antigen detection system: the plate was coated with rabbit anti-CA16 polyclonal antibody, placed at 2-8°C overnight, blocked for 1-2 hours with 10% fetal bovine serum at 37°C, and added virus culture that to be identified. A negative control well was set up. The plate was then incubated at 36~37°C for 1 hour, washed for 3~5 times, added with a CA16 specific monoclonal antibody, incubated at 36~37°C for 1 hour, washed for 3~5 times, and colored, and then the reaction was terminated.

CA10 antigen detection system: the plate was coated with rabbit anti-CA10 polyclonal antibody, placed at 2-8°C overnight, blocked for 1-2 hours with 10% fetal bovine serum at 37°C, and added virus culture that to be identified. A negative control wells was set up. The plate was then incubated at 36~37°C for 1 hour, washed for 3~5 times, added with a CA10 specific monoclonal antibody, incubated at 36~37°C for 1 hour, washed for 3~5 times, and colored, and then the reaction was terminated.

CA6 antigen detection system: the plate was coated with rabbit anti-CA6 polyclonal antibody, placed at 2-8°C overnight, blocked for 1-2 hours with 10% fetal bovine serum at 37°C, and added virus culture that to be identified. A negative control well was set up. The plate was then incubated at 36~37°C for 1 hour, washed for 3~5 times, added with a CA6 specific monoclonal antibody, incubated at 36~37°C for 1 hour, washed for 3~5 times, and colored, and then the reaction was terminated.

The above four antigen detection systems were used to identify the screened CA16, CA10, CA6 and EV71 strains, and the results were shown in Table 1 to Table 4.

Table 1. Identification results of Elisa antigenic system of CA16 strain

| Strain | OD value | Results |
|---|---|---|
| CA16 strain | 2.015 | Positive |
| CA10 strain | 0.054 | Negative |
| CA6 strain | 0.061 | Negative |
| EV71 strain | 0.049 | Negative |
| Negative control well | 0.068 | Negative is established |

Table 2. Identification results of Elisa antigenic system of CA10 strain

| Strain | OD value | Results |
|---|---|---|
| CA16 strain | 0.034 | Negative |
| CA10 strain | 1.875 | Positive |
| CA6 strain | 0.045 | Negative |
| EV71 strain | 0.056 | Negative |
| Negative control well | 0.058 | Negative is established |

Table 3. Identification results of Elisa antigenic system of CA6 strain

| Strain | OD value | Results |
|---|---|---|
| CA16 strain | 0.042 | Negative |
| CA10 strain | 0.059 | Negative |
| CA6 strain | 2.154 | Positive |
| EV71 strain | 0.061 | Negative |
| Negative control well | 0.047 | Negative is established |

Table 4. Identification results of Elisa antigenic system of EV71 strain

| Strain | OD value | Results |
|---|---|---|
| CA16 strain | 0.039 | Negative |

(continued)

| Strain | OD value | Results |
|---|---|---|
| CA10 strain | 0.043 | Negative |
| CA6 strain | 0.038 | Negative |
| EV71 strain | 2.046 | Positive |
| Negative control well | 0.050 | Negative is established |

Molecular level identification: virus RNA was extracted by a kit (QIAGEN), one-step reverse transcription by reverse transcription kit (TAKARA) and PCR amplification of the extracted RNA were performed, primer sequences were designed by the present inventor and synthesized by BGI Sequencing Co., Ltd., and PCR products were identified by 1% agarose gel electrophoresis. Reverse transcription conditions are as follows:

Reverse transcription reaction:      45°C for 10 min
Reverse transcriptase inactivation:      94°C for 2 min

Denature: 98°C for 10s

Annealing: 50-65°C for 15s     }   30 cycles

Extension: 68-72°C 10s/kb

After PCR amplification, sequencing, and comparison of sequencing results in NCBI database, the corresponding types of CA16, CA10, and CA6 strains were identified.

## (3) Production of monovalent stock solution

Microcarrier of 2~6 g/L was fed into a 130L fermenter, and Vero cells were seeded in an amount of $10\sim50\times10^4$ cells/mL, with a pH of 7.0-7.5, and the dissolved oxygen content fluctuating within a small range of 50%. The cells were cultured at 35~37.5°C for 3~7 days, inoculated with the virus at a ratio of MOI 0.001-0.1 according to the number of cells, and further cultured for 3-6 days before the virus was harvested.

The virus was cleared through a 100-500KD of ultrafiltration membrane package, ultrafiltered and concentrated to remove impurity proteins. The ultrafiltered and concentrated virus suspension was purified by sucrose density gradient centrifugation to remove impurities and separate hollow and solid virus particles, and the ultracentrifugation conditions were 2-8°C, the centrifugation speed was 20000-50000rpm, and the centrifugation time was 8-18 hours. The ultrafiltered and centrifugated target tube virus was collected and desugared by ultrafiltration. The desugared virus suspension was treated by stirring with a non-restriction endonuclease at 18-30°C, and ultrafiltered to remove the host cell DNA. A formaldehyde solution of 1:2000-1:8000 was used to inactivate the virus at 35-38°C for 2-6 days until the virus was completely inactivated, and after the above steps, the virus stock solutions of CA16, CA10, and CA6 types were obtained, respectively.

The EV71 stock solution was produced by culturing in a fermenters or a cell factory, and the virus was harvested and then inactivated and purified, and the other process stages were comparable to that of CA16, CA10 and CA6 types.

## (4) Preparation of monovalent aluminum adsorption vaccine

Preparation of monovalent aluminum adsorption vaccine: aluminum adjuvant was diluted to 3.0mg/mL (calculated by $Al(OH)_3$) with 0.85% normal saline; EV71 antigen was diluted to 400-6000U/mL, CA16 and CA10 antigens to 800U/mL-9600U/mL respectively, CA6 antigen to 4000 U/mL-18000U/mL, with 0.01M PBS, and then the diluted aluminum adjuvants were added to the diluted EV71, CA16, CA10 and CA6 antigens respectively at room temperature. The aluminum adjuvant and the antigen were added in equal volumes to perform adsorption, stirred while adding and after addition for 30 minutes at room temperature to obtain EV71, CA16, CA10 and CA6 monovalent aluminum adsorption vaccines.

**(5) Preparation of quadrivalent combination vaccine**

The above four types of monovalent aluminum adsorption products were mixed in a certain proportion and then packaged to prepare a finished quadrivalent combination vaccine for HFMD. The specific operation process is as follows: The EV71, CA16, CA10 and CA6 monovalent aluminum adsorption vaccines obtained above were mixed in a certain proportion, and appropriately diluted with a 0.01M PBS (pH 7.2) solution and aluminum hydroxide solution according to the antigen content of the vaccine stock solution, and then stirred and adsorbed at room temperature for $20\pm10$ minutes to provide the semi-finished vaccine. In the obtained semi-finished vaccine, the final concentration of aluminum hydroxide was about 1.30mg/mL (0.45mg/mL if calculated by aluminum ions), the antigen content of EV71 was 960U/mL, the antigen content of CA16 was 200 U/mL~1600U/mL, the antigen content of CA10 was 200 U/mL~1600U/mL, and the antigen content of CA6 was 1000U/mL~3000U/mL. The protein doses corresponding to the above antigen contents in the semi-finished product ranged from 1μg/mL~8μg/mL, respectively, that is, the total protein dose of the quadrivalent semi-finished vaccine was 4 μg/mL~32μg/mL. After that, the semi-finished vaccine was filled in a syringe to obtain the finished combination vaccine. The dose of the finished combination vaccine was 0.5mL/person, and the protein content of each virus component in the finished combination vaccine was ≤4μg/person.

Quadrivalent combination vaccine for HFMD was prepared according to the above method, and the specific parameters were shown in Table 5, and three consecutive batches of combination vaccine were prepared for each parameter.

Table 5. Parameter settings of quadrivalent combination vaccine

| | Content of antigen | | | |
| --- | --- | --- | --- | --- |
| | EV71/(U/mL) | CA16/(U/mL) | CA10/(U/mL) | CA6/(LT/mL) |
| Example 1 | 960 | 800 | 200 | 2000 |
| Example 2 | 960 | 800 | 500 | 2000 |
| Example 3 | 960 | 800 | 800 | 2000 |
| Example 4 | 960 | 800 | 1000 | 2000 |
| Example 5 | 960 | 800 | 1300 | 2000 |
| Example 6 | 960 | 800 | 1600 | 2000 |
| Example 7 | 960 | 800 | 800 | 1000 |
| Example 8 | 960 | 800 | 800 | 1500 |
| Example 9 | 960 | 800 | 800 | 2500 |
| Example 10 | 960 | 800 | 800 | 3000 |
| Example 11 | 960 | 200 | 800 | 2000 |
| Example 12 | 960 | 500 | 800 | 2000 |
| Example 13 | 960 | 1000 | 800 | 2000 |
| Example 14 | 960 | 1300 | 800 | 2000 |
| Example 15 | 960 | 1600 | 800 | 2000 |

**2. Evaluation of the combination vaccine**

**(1) Test of the finished quadrivalent combination vaccine**

The contents of aluminum hydroxide, supernatant antigen and dissociated antigen in the quadrivalent combination vaccine for HFMD from Examples 1 to 15 above were detected respectively, and the adsorption rate and dissociation rate were calculated. The results are shown in Table 6.

Table 6. Test results of the finished quadrivalent combination vaccine

| | Batch | Content of aluminum hydroxide (mg/mL) | EV71 antigen component | | CA16 antigen component | | CA10 antigen component | | CA6 antigen component | |
|---|---|---|---|---|---|---|---|---|---|---|
| | | | adsorption/ % | dissociation/ % | adsorption/ % | dissociation/ % | adsorption/ % | dissociation/ % | adsorption/ % | dissociation/ % |
| Example 1 | 1 | 1.31 | >99 | 100 | 98 | 106 | >99 | 99 | >99 | 97 |
| | 2 | 1.31 | >99 | 99 | 97 | 95 | >99 | 89 | >99 | 93 |
| | 3 | 1.30 | >99 | 99 | >99 | 112 | >99 | 95 | >99 | 97 |
| Example 2 | 1 | 1.31 | >99 | 112 | 97 | 88 | >99 | 94 | >99 | 97 |
| | 2 | 1.32 | >99 | 91 | 97 | 100 | >99 | 90 | >99 | 98 |
| | 3 | 1.28 | >99 | 89 | >99 | 112 | >99 | 94 | >99 | 92 |
| Example 3 | 1 | 1.31 | >99 | 101 | 97 | 99 | >99 | 92 | >99 | 90 |
| | 2 | 1.27 | >99 | 95 | 99 | 100 | >99 | 90 | >99 | 110 |
| | 3 | 1.28 | >99 | 95 | 99 | 96 | >99 | 97 | >99 | 95 |
| Example 4 | 1 | 1.29 | >99 | 94 | >99 | 111 | >99 | 93 | >99 | 92 |
| | 2 | 1.29 | >99 | 101 | 99 | 93 | >99 | 95 | >99 | 108 |
| | 3 | 1.27 | >99 | 101 | >99 | 93 | >99 | 97 | >99 | 96 |
| Example 5 | 1 | 1.32 | >99 | 96 | >99 | 106 | >99 | 90 | >99 | 101 |
| | 2 | 1.31 | >99 | 111 | 99 | 96 | >99 | 93 | >99 | 106 |
| | 3 | 1.28 | >99 | 106 | 98 | 101 | >99 | 97 | >99 | 89 |
| Example 6 | 1 | 1.28 | >99 | 95 | 98 | 102 | >99 | 89 | >99 | 96 |
| | 2 | 1.27 | >99 | 110 | 98 | 101 | >99 | 94 | >99 | 106 |
| | 3 | 1.29 | >99 | 88 | 98 | 99 | >99 | 102 | >99 | 92 |
| Example 7 | 1 | 1.30 | >99 | 96 | >99 | 102 | >99 | 90 | >99 | 99 |
| | 2 | 1.30 | >99 | 114 | >99 | 101 | >99 | 95 | >99 | 92 |
| | 3 | 1.28 | >99 | 95 | 96 | 98 | >99 | 94 | >99 | 98 |

(continued)

| | Batch | Content of aluminum hydroxide (mg/mL) | EV71 antigen component | | CA16 antigen component | | CA10 antigen component | | CA6 antigen component | |
|---|---|---|---|---|---|---|---|---|---|---|
| | | | adsorption/% | dissociation/% | adsorption/% | dissociation/% | adsorption/% | dissociation/% | adsorption/% | dissociation/% |
| Example 8 | 1 | 1.32 | >99 | 103 | >99 | 98 | >99 | 86 | >99 | 100 |
| | 2 | 1.30 | >99 | 98 | >99 | 102 | >99 | 95 | >99 | 110 |
| | 3 | 1.25 | >99 | 101 | 98 | 96 | >99 | 94 | >99 | 98 |
| Example 9 | 1 | 1.31 | >99 | 99 | 98 | 95 | >99 | 93 | >99 | 95 |
| | 2 | 1.28 | >99 | 96 | 99 | 96 | >99 | 92 | >99 | 96 |
| | 3 | 1.29 | >99 | 93 | 98 | 97 | >99 | 93 | >99 | 98 |
| Example 10 | 1 | 1.27 | >99 | 100 | >99 | 99 | >99 | 90 | >99 | 95 |
| | 2 | 1.29 | >99 | 101 | >99 | 99 | >99 | 100 | >99 | 95 |
| | 3 | 1.30 | >99 | 93 | 98 | 97 | >99 | 90 | >99 | 92 |
| Example 11 | 1 | 1.31 | >99 | 99 | 98 | 95 | >99 | 95 | >99 | 99 |
| | 2 | 1.32 | >99 | 98 | 98 | 110 | >99 | 98 | >99 | 101 |
| | 3 | 1.29 | >99 | 110 | >99 | 87 | >99 | 99 | >99 | 101 |
| Example 12 | 1 | 1.30 | >99 | 111 | 98 | 100 | >99 | 96 | >99 | 102 |
| | 2 | 1.31 | >99 | 89 | 98 | 96 | >99 | 87 | >99 | 100 |
| | 3 | 1.28 | >99 | 106 | 99 | 98 | >99 | 93 | >99 | 99 |
| Example 13 | 1 | 1.27 | >99 | 104 | >99 | 102 | >99 | 95 | >99 | 89 |
| | 2 | 1.27 | >99 | 101 | 99 | 99 | >99 | 96 | >99 | 105 |
| | 3 | 1.27 | >99 | 88 | 97 | 94 | >99 | 102 | >99 | 92 |
| Example 14 | 1 | 1.28 | >99 | 104 | 99 | 87 | >99 | 91 | >99 | 90 |
| | 2 | 1.26 | >99 | 102 | 97 | 99 | >99 | 92 | >99 | 95 |
| | 3 | 1.28 | >99 | 99 | 99 | 102 | >99 | 94 | >99 | 111 |

(continued)

| | Batch | Content of aluminum hydroxide (mg/mL) | EV71 antigen component | | CA16 antigen component | | CA10 antigen component | | CA6 antigen component | |
|---|---|---|---|---|---|---|---|---|---|---|
| | | | adsorption/ % | dissociation/ % | adsorption/ % | dissociation/ % | adsorption/ % | dissociation/ % | adsorption/ % | dissociation/ % |
| Example 15 | 1 | 1.30 | >99 | 105 | 99 | 101 | >99 | 89 | >99 | 90 |
| | 2 | 1.29 | >99 | 100 | 97 | 93 | >99 | 92 | >99 | 93 |
| | 3 | 1.29 | >99 | 101 | >99 | 102 | >99 | 98 | >99 | 108 |

**(2) Immunogenicity test**

Rats and mice were immunized with two doses of the three batches of quadrivalent combination vaccine in Example 3 respectively to detect the titers of neutralizing antibodies in serum. Monovalent control and negative control were set up simultaneously and repeated three times. The neutralizing antibody titer was detected by microcytopathic assay. The cells for the assay were RD cells, and the cells for the neutralization experiment were cultured at 37°C for 7 days, and the cytopathic changes were observed. The results of neutralizing antibody titer and positive conversion rate are shown in Table 7. The immunogenicity of each virus component in the combination vaccine was statistically analyzed with the corresponding monovalent vaccine, and the results are shown in Table 8. There was no significant difference in GMT values of neutralizing antibodies between the combination vaccine and single vaccine according to statistical analysis (All P values were greater than 0.05).

Table 7. Neutralizing antibody titer and positive conversion rate

| Batch | EV71 | | CA16 | | CA10 | | CA6 | |
|---|---|---|---|---|---|---|---|---|
| | Neutralizing antibodies GMT (1: ) | Positive conversion rate (%) | Neutralizing antibodies GMT (1: ) | Positive conversion rate (%) | Neutralizing antibodies GMT (1: ) | Positive conversion rate (%) | Neutralizing antibodies GMT (1: ) | Positive conversi on rate (%) |
| Single vaccine | 414 | 100 | 138 | 100 | 389 | 100 | 366 | 100 |
| 1 | 426 | 100 | 154 | 100 | 414 | 100 | 398 | 100 |
| 2 | 440 | 100 | 144 | 100 | 436 | 100 | 432 | 100 |
| 3 | 468 | 100 | 168 | 100 | 406 | 100 | 428 | 100 |

Table 8. Statistical analysis of immunogenicity between combination vaccine and single vaccine

| Batch | P value | | | |
|---|---|---|---|---|
| | EV71 | CA16 | CA10 | CA6 |
| 1 | >0.05 | >0.05 | >0.05 | >0.05 |
| 2 | >0.05 | >0.05 | >0.05 | >0.05 |
| 3 | >0.05 | >0.05 | >0.05 | >0.05 |

**(3) Safety evaluation**

During the entire experimental period in which the immunogenicity of each combination vaccine was evaluated, all animals were healthy and survived, and there were no abnormalities in clinical performance. Three batches of EV71/CA16/CA10/CA6 quadrivalent combination vaccines in Example 3 were evaluated for animal safety, including abnormal toxicity test, mouse acute toxicity test and guinea pig active allergy test.

Abnormal toxicity tests included mouse test and guinea pig test. In the mouse test, the mouse was injected with 0.5mL EV71/CA16/CA10/CA6 combination vaccine, 5 mice in total, 18~22g each, and observed for 7d. During the observation period, all the mice survived without abnormal reactions, and the mice gained weight at the end of the test. In the guinea pig test, the guinea pig was injected with 5mL EV71/CA16/CA10/CA6 combination vaccine, 2 guinea pigs in total, 250~350g each, and observed for 7d. During the observation period, all the guinea pigs survived without abnormal reactions, and the guinea pigs gained weight at the end of the test.

In the mouse acute toxicity test, the EV71/CA16/CA10/CA6 combination vaccine was administered intramuscularly to a Kunming mouse at its maximum dose of 8000U/kg body weight (equivalent to about 120 times the clinical dose for a child of 0.5 years old), and the administered animals did not have abnormal clinical symptoms or death.

In the guinea pig active allergy test, a Hartley guinea pig was given the combination vaccine 3 times by intramuscular injection for sensitization at a dose of 0.5mL/pc/time, and administered every other day. Intravenous injection for provocation was performed once on Day 14 after the last sensitization administration at a dose of 1.0mL/pc/time. The test animals were continuously observed for allergic reactions after the provocation administration, and the test results showed that the EV71/CA16/CA10/CA6 combination vaccines had an negative allergic reaction in the guinea pig systemic active allergy test.

**(4) Stability evaluation**

The three batches of the finished combination vaccine prepared in Example 3 were stored under an environment of 2~8°C, and the antigen content, appearance, capacity, pH value, osmolarity molarity, bacterial endotoxin, formaldehyde content, etc. were detected by sampling at the indicated time point. Data were recorded up to 12 months, and the vaccine was detected for abnormal toxicity and immunogenicity in the 12th month. The finished combination vaccine was placed at 2-8°C for 12 months, and the detection indicators did not decrease significantly. The results are shown in Table 9~Table 11. Table 9. Test results of antigen content of the finished combination vaccine at 2~8°C (percentage of labeled amount)

| Component | Batch | Content of antigen (%) at the time point (month) | | |
|---|---|---|---|---|
| | | 0 | 6 | 12 |
| EV71 | 1 | 101 | 98 | 97 |
| | 2 | 104 | 105 | 102 |
| | 3 | 98 | 102 | 99 |
| CA16 | 1 | 102 | 98 | 101 |
| | 2 | 105 | 103 | 102 |
| | 3 | 101 | 96 | 97 |

(continued)

| Component | Batch | Content of antigen (%) at the time point (month) | | |
|---|---|---|---|---|
| | | 0 | 6 | 12 |
| CA10 | 1 | 103 | 107 | 105 |
| | 2 | 100 | 103 | 104 |
| | 3 | 103 | 104 | 101 |
| CA6 | 1 | 101 | 101 | 98 |
| | 2 | 100 | 105 | 99 |
| | 3 | 99 | 102 | 102 |

Table 10. Test result 1 of the finished combination vaccine at 2~8°C

| Batch | Storage time (month) | pH value | Osmolarity molarity (mOsmol/kg) | Sterility test |
|---|---|---|---|---|
| | / | 6.0-7.5 | 245-310 | No bacterial growth |
| 1 | 0 | 7.1 | 285 | No bacterial growth |
| | 6 | 7.2 | 284 | No bacterial growth |
| | 12 | 7.2 | 285 | No bacterial growth |
| 2 | 0 | 7.2 | 278 | No bacterial growth |
| | 6 | 7.1 | 275 | No bacterial growth |
| | 12 | 7.2 | 280 | No bacterial growth |
| 3 | 0 | 7.2 | 274 | No bacterial growth |
| | 6 | 7.2 | 270 | No bacterial growth |
| | 12 | 7.1 | 272 | No bacterial growth |

Table 11. Test result 2 of the finished combination vaccine at 2~8°C

| Batch | Storage time (month) | Abnormal toxicity | Bacterial endotoxin (EU/mL) | Immunogenicity neutralizing antibody GMT (1: ) | | | |
|---|---|---|---|---|---|---|---|
| | / | Should conform to regulations | <5 | EV71 | CA16 | CA10 | CA6 |
| 1 | 0 | conform to regulations | <5 | 426 | 154 | 414 | 398 |
| | 6 | / | <5 | / | / | / | / |
| | 12 | conform to regulations | <5 | 418 | 138 | 390 | 412 |
| 2 | 0 | conform to regulations | <5 | 440 | 144 | 436 | 432 |
| | 6 | / | <5 | / | / | / | / |
| | 12 | conform to regulations | <5 | 458 | 162 | 420 | 440 |

(continued)

| Batch | Storage time (month) | Abnormal toxicity | Bacterial endotoxin (EU/mL) | Immunogenicity neutralizing antibody GMT (1: ) | | | |
|---|---|---|---|---|---|---|---|
| | / | Should conform to regulations | <5 | EV71 | CA16 | CA10 | CA6 |
| 3 | 0 | conform to regulations | <5 | 468 | 168 | 406 | 428 |
| | 6 | / | <5 | / | / | / | / |
| | 12 | conform to regulations | <5 | 482 | 174 | 389 | 416 |
| Note: "/" represents that the test is not performed. | | | | | | | |

In addition to Example 3, Examples 1-2 and Examples 4-15 were also tested for immunogenicity, and safety evaluation and stability evaluation were also carried out. The results showed that there was no significant difference between the GMT value of the neutralizing antibody of single vaccine, and that of the combination vaccines in these examples. Further, similar to Example 3, the combination vaccines in these examples all had the advantages of good safety and high stability.

The preferred embodiment of the present invention is described in details above; however, the present invention is not limited to the specific details in the above embodiments. Within the scope of the technical concept of the present invention, a variety of simple variations of the technical solution of the present invention may be carried out, and these simple variations belong to the scope of protection of the present invention.

It should also be noted that each specific technical feature described in the above embodiment, without contradiction, may be combined in any suitable way. In order to avoid unnecessary repeating, the present invention will not be otherwise described for various possible combinations.

In addition, different embodiments in the present invention can be arbitrarily combined. As long as the combination does not contradict the concept of the present invention, it should also be regarded as the content disclosed by the present invention.

## Claims

1. A combination vaccine for preventing HFMD, wherein the combination vaccine comprises inactivated Enterovirus type 71, and inactivated Coxsackievirus A group type 16, type 10 and type 6.

2. The combination vaccine according to claim 1, wherein the deposit number of Coxsackievirus A group type 16 strain is CGMCC No. 18886.

3. The combination vaccine according to claim 1, wherein the deposit number of Coxsackievirus A group type 10 strain is CGMCC No. 18887.

4. The combination vaccine according to claim 1, wherein the deposit number of Coxsackievirus A group type 6 strain is CGMCC No. 18888.

5. The combination vaccine according to any one of claims 1-4, wherein a content of antigen of the inactivated Enterovirus type 71 in the combination vaccine is 100~1000U/mL;

   a content of antigen of the inactivated Coxsackievirus A group type 16 in the combination vaccine is 200~3000U/mL, preferably is 200~1600U/mL;
   a content of antigen of the inactivated Coxsackievirus A group type 10 in the combination vaccine is 200~3000U/mL, preferably is 200~1600U/mL; and
   a content of antigen of the inactivated Coxsackievirus A group type 6 in the combination vaccine is 1000~3000U/mL.

6. The combination vaccine according to any one of claims 1-5, wherein the combination vaccine further comprises an aluminum adjuvant;

    preferably, the aluminum adjuvant is selected from the group consisting of aluminum hydroxide, aluminum phosphate and aluminum sulfate; and
    more preferably, the aluminum adjuvant is aluminum hydroxide.

7. The combination vaccine according to claim 6, wherein the final concentration of the aluminum adjuvant in aluminum content calculated by aluminum ion is 0.1-1.0 mg/mL, preferably 0.2-0.8 mg/mL.

8. The combination vaccine according to any one of claims 1-7, wherein the combination vaccine is in liquid form.

9. A method for the preparation of the combination vaccine according to any one of claims 1-8, comprising the following steps preparing a stock solution for each of the viruses respectively, using an aluminum adjuvant to adsorb each of the stock solutions respectively to provide a virus aluminum adsorption product, and then mixing the aluminum adsorption products to provide the combination vaccine.

10. Use of the combination vaccine according to any one of claims 1-8 in the preparation of a medicament for the prevention or treatment of a disease caused by Enterovirus type 71, Coxsackievirus A group type 16, Coxsackievirus A group type 10, and/or Coxsackievirus A group type 6.

## INTERNATIONAL SEARCH REPORT

International application No.

**PCT/CN2020/141693**

### A. CLASSIFICATION OF SUBJECT MATTER

A61K 39/125(2006.01)i; A61K 39/39(2006.01)i; A61P 31/14(2006.01)i

According to International Patent Classification (IPC) or to both national classification and IPC

### B. FIELDS SEARCHED

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

VEN, CNABS, CNPTXT, EPTXT, USTXT, WOTXT, ISI Web of Knowledge, CNKI, NCBI GENBANK: 科兴, 柯萨奇病毒, 肠道病毒, 疫苗, 联合疫苗, 四价疫苗, 多价疫苗, 手足口病, 灭活, CGMCC, E71, A6, A16, A10, 18888, 18887, 18886, Vaccine, HFMD, Coxsackievirus, Enterovirus, SINOVAC, Inactivated, Multivalent

### C. DOCUMENTS CONSIDERED TO BE RELEVANT

| Category* | Citation of document, with indication, where appropriate, of the relevant passages | Relevant to claim No. |
|---|---|---|
| Y | CN 105963692 A (SINOVAC BIOTECH CO., LTD.) 28 September 2016 (2016-09-28) see abstract, claims 1-2, 4, 9-10, description paragraphs 31, 143-155 | 1-10 |
| Y | LIM, H. et al. "The immunogenicity and protection effect of an inactivated coxsackievirus A6, A10, and A16 vaccine against hand, foot, and mouth disease" *VACCINE*, Vol. 36, No. 24, 07 June 2018 (2018-06-07), see abstract, figure 7 | 1-10 |
| Y | CN 106075423 A (SINOVAC BIOTECH CO., LTD.) 09 November 2016 (2016-11-09) see abstract, claims 1, 4 | 1-10 |
| Y | CAINE, E.A. et al. "Efficacy of a Trivalent Hand, Foot, and Mouth Disease Vaccine against Enterovirus 71 and Coxsackieviruses A16 and A6 in Mice" *VACCINE*, Vol. 7, No. 11, 17 November 2015 (2015-11-17), see abstract | 1-10 |
| A | WO 2010139193 A1 (SINOVAC BIOTECH CO., LTD.) 09 December 2010 (2010-12-09) entire document | 1-10 |

☑ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **13 September 2021** | **28 September 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**EP 4 272 755 A1**

| International application No. |
|---|
| **PCT/CN2020/141693** |

**C.    DOCUMENTS CONSIDERED TO BE RELEVANT**

| Category* | Citation of document, with indication, where appropriate, of the relevant passages | Relevant to claim No. |
|---|---|---|
| A | CN 101695570 A (INSTITUTE OF MICROBIOLOGY AND EPIDEMIOLOGY, PLA ACADEMY OF MILITARY MEDICAL SCIENCE) 21 April 2010 (2010-04-21)<br>entire document | 1-10 |
| A | CN 101780278 A (FORWELL BIOPHARMACEUTICALS CO., LTD.) 21 July 2010 (2010-07-21)<br>entire document | 1-10 |
| A | CN 103386126 A (SINOVAC BIOTECH CO., LTD.) 13 November 2013 (2013-11-13)<br>entire document | 1-10 |
| A | CN 105999256 A (SINOVAC BIOTECH CO., LTD.) 12 October 2016 (2016-10-12)<br>entire document | 1-10 |
| A | CN 107739731 A (TAISHAN MEDICAL UNIVERSITY) 27 February 2018 (2018-02-27)<br>entire document | 1-10 |
| A | CN 108853490 A (INSTITUTE OF MEDICAL BIOLOGY, CHINESE ACADEMY OF MEDICAL SCIENCES) 23 November 2018 (2018-11-23)<br>entire document | 1-10 |

Form PCT/ISA/210 (second sheet) (January 2015)

# EP 4 272 755 A1

**INTERNATIONAL SEARCH REPORT**
**Information on patent family members**

International application No.

**PCT/CN2020/141693**

| Patent document cited in search report | | | Publication date (day/month/year) | Patent family member(s) | | | Publication date (day/month/year) |
|---|---|---|---|---|---|---|---|
| CN | 105963692 | A | 28 September 2016 | CN | 105963692 | B | 09 February 2021 |
| CN | 106075423 | A | 09 November 2016 | CN | 106075423 | B | 06 December 2019 |
| WO | 2010139193 | A1 | 09 December 2010 | CN | 101575593 | A | 11 November 2009 |
| | | | | CN | 101575593 | B | 04 July 2012 |
| CN | 101695570 | A | 21 April 2010 | CN | 101695570 | B | 27 August 2014 |
| CN | 101780278 | A | 21 July 2010 | CN | 101780278 | B | 01 February 2012 |
| CN | 103386126 | A | 13 November 2013 | CN | 103386126 | B | 17 June 2015 |
| CN | 105999256 | A | 12 October 2016 | CN | 105999256 | B | 22 January 2021 |
| CN | 107739731 | A | 27 February 2018 | CN | 107739731 | B | 21 July 2020 |
| CN | 108853490 | A | 23 November 2018 | | None | | |